(19)

**Europäisches Patentamt**
**European Patent Office**
**Office européen des brevets**

(11) **EP 4 585 904 A1**

(12) **EUROPEAN PATENT APPLICATION**
published in accordance with Art. 153(4) EPC

(43) Date of publication:
**16.07.2025 Bulletin 2025/29**

(51) International Patent Classification (IPC):
*G01N 17/00* (2006.01)  *G01N 3/56* (2006.01)
*G01M 13/04* (2019.01)  *G01N 33/30* (2006.01)

(21) Application number: **23891130.9**

(22) Date of filing: **30.08.2023**

(52) Cooperative Patent Classification (CPC):
**G01M 13/04; G01N 3/56; G01N 17/00; G01N 33/30**

(86) International application number:
**PCT/JP2023/031576**

(87) International publication number:
**WO 2024/105971 (23.05.2024 Gazette 2024/21)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **14.11.2022 JP 2022182065**

(71) Applicant: **NSK LTD.**
**Tokyo 141-8560 (JP)**

(72) Inventors:
• **ENAMI, Kakeru**
**Fujisawa-shi, Kanagawa 251-8501 (JP)**
• **YAMADA, Hiroki**
**Fujisawa-shi, Kanagawa 251-8501 (JP)**
• **KOMATA, Hiroki**
**Fujisawa-shi, Kanagawa 251-8501 (JP)**

(74) Representative: **Grünecker Patent- und
Rechtsanwälte
PartG mbB
Leopoldstraße 4
80802 München (DE)**

(54) **METHOD FOR PREDICTING AMOUNT OF HYDROGEN GENERATED IN ROLLING DEVICE, HYDROGEN GENERATION TEST DEVICE USED IN SAID METHOD, AND METHOD FOR EVALUATING LIKELIHOOD OF WHITE TISSUE PEELING OCCURRING IN ROLLING DEVICE**

(57)  A method for predicting an amount of hydrogen generated in a rolling device includes: a step in which, while two sliding members are caused to slide against each other over a contact surface with a lubricant interposed between the two sliding members, an exposed area of a newly-generated surface due to direct contact between the two sliding members is calculated, an amount of hydrogen generated from the lubricant is measured, and a correlation between the exposed area of the newly-generated surface and the amount of hydrogen generated is obtained, in which the exposed area of the newly-generated surface is calculated using a metal contact ratio and a sliding speed of the two sliding members.

**Fig.1**

**Description**

**Technical Field**

The present invention relates to a method for predicting an amount of hydrogen generated in a rolling device, a hydrogen generation test device used in the method, and a method for evaluating likelihood of white structure peeling occurring in a rolling device.

**Background Art**

White structure peeling is one of the peeling modes of a rolling device caused by hydrogen entering a metal member of the rolling device such as a bearing, and is a problem because peeling occurs earlier than a calculated life. The origin of hydrogen is considered to be a lubricant used to lubricate the rolling device. It is said that the lubricant is decomposed and hydrogen is generated during use of the rolling device. When the use conditions of the rolling device and the amount of hydrogen generated from the lubricant can be quantitatively evaluated, it is considered that the likelihood of white structure peeling under the use conditions can be predicted. However, since hydrogen is easily diffused, it is difficult to measure the amount of hydrogen generated in the rolling device as an actual machine. Therefore, it is not clear how much hydrogen is generated from the lubricant under what use conditions.

Therefore, the amount of hydrogen generated is evaluated using an element testing machine instead of the rolling device (actual machine) such as a bearing. For example, Patent Literature 1 below discloses an evaluation device capable of evaluating diffusible hydrogen entering components constituting the rolling device such as a bearing. This evaluation device uses a testing machine that presses a rotating disk-shaped sliding member against a disk-shaped test piece to slide, and detects diffusible hydrogen that enters the test piece under a pure sliding condition and is released into a diffusible hydrogen detection chamber by vacuuming.

**Citation List**

**Patent Literature**

Patent Literature 1: Japanese Unexamined Patent Publication No. 2013-234883

**Summary of Invention**

**Technical Problem**

However, the evaluation device described in Patent Literature 1 can evaluate the hydrogen generation amount only under a pure sliding condition, and there is a possibility that it cannot realize contact conditions that occur in the rolling device as an actual machine, that is, a pure rolling condition and a rolling sliding condition. Therefore, in this evaluation device, an amount of hydrogen generated in the actual machine cannot be predicted from test conditions.

The present invention has been made in view of the above problem, and an object thereof is to provide a method for predicting an amount of hydrogen generated in a rolling device capable of predicting an amount of hydrogen generated from a lubricant in a rolling device, a hydrogen generation test device used in the method, and a method for evaluating likelihood of white structure peeling occurring in a rolling device.

**Solution to Problem**

The present inventors have conducted studies to solve the above problem. Specifically, the present inventors measured the amount of hydrogen generated from the lubricant while changing test conditions while causing two sliding members having the lubricant interposed therebetween to slide against each other over a contact surface. As a result, the present inventors have found that when only a metal contact ratio between the two sliding members is increased as the test conditions, the amount of hydrogen generated from the lubricant tends to increase with the increase in the metal contact ratio. Here, the metal contact ratio is a ratio of an area of an actual contact surface to an area of an apparent contact surface of the two sliding members. Here, the apparent contact surface represents a portion where the surfaces of the two sliding members are in macroscopic contact. In addition, surface uneven portions exist on the surface of the actual sliding member. Therefore, actually, the contact between the two surfaces (the surfaces of the two sliding members) is performed by protrusions (convex portions) of the surface uneven portions of the two surfaces. In the apparent contact surface, a portion where the protrusions of the surface uneven portions are in direct contact with each other is the actual contact surface. In addition, the present inventors have found that when only the sliding speed of the two sliding members is increased as the test conditions, the amount of hydrogen generated from the lubricant tends to increase with the increase in the sliding speed. Here, the sliding speed is a difference in circumferential speed between the two sliding members. The sliding speed can also realize the pure rolling condition or the rolling sliding condition in the rolling device between the two sliding members. For example, when the circumferential speeds of the two sliding members are equal, the sliding speed is 0, and the condition in the rolling device is a pure rolling condition. When the circumferential speeds of the two sliding members are different from each other in a state where the two sliding members are rotating, the condition in the rolling device is a rolling sliding condition. In response to the above results, the present inventors have further conducted intensive studies, and found that a good correlation can be obtained between the amount of hydrogen generated from the lubricant and an exposed area of a newly-generated surface, which is a parameter calculated using the metal contact ratio and the sliding speed of the two sliding members. The present inventors have found that the above problem can be solved by the following invention. The newly-generated surface refers to a surface of metal exposed by removing an oxide film in the sliding members when the two sliding members slide in contact with each other, and the exposed area of the newly-generated surface refers to an area of the newly-generated surface.

That is, one aspect of the present invention provides a method for predicting an amount of hydrogen generated in a rolling device, the method including: a step in which, while two sliding members having a lubricant interposed therebetween are caused to slide against each other over a contact surface, an exposed area of a newly-generated surface due to direct contact between the two sliding members is calculated, an amount of hydrogen generated from the lubricant is measured, and a correlation between the exposed area of the newly-generated surface and the amount of hydrogen generated is obtained, in which the exposed area of the newly-generated surface is calculated using a metal contact ratio and a sliding speed of the two sliding members.

According to the method for predicting an amount of hydrogen generated, the exposed area of the newly-generated surface is calculated using the sliding speed, and the sliding speed can realize a pure rolling condition or a rolling sliding condition in the rolling device between the two sliding members. Therefore, the amount of hydrogen generated from the lubricant can reflect the amount of hydrogen generated in the rolling device. Therefore, when the exposed area of the newly-generated surface is the exposed area of the newly-generated surface in the rolling device, the amount of hydrogen generated in the rolling device can be predicted from the correlation.

Another aspect of the present invention provides a method for predicting an amount of hydrogen generated in a rolling device, the method including: a first step in which, while two sliding members having a lubricant interposed therebetween are caused to slide against each other over a contact surface, an exposed area of a newly-generated surface due to direct contact between the two sliding members is calculated; and a second step in which a hydrogen generation amount $\Delta H1$ due to sliding is calculated based on the exposed area of the newly-generated surface and the following Formula (1), in which the exposed area of the newly-generated surface is calculated using a metal contact ratio and a sliding speed of the two sliding members.

$$\Delta H1/S = B \times \exp(-Ea/RT)...\ (1)$$

(In the Formula (1), B represents a constant, S represents an exposed area ($m^2$) of the newly-generated surface, Ea represents activation energy (J/mol) of the lubricant in a reaction with the newly-generated surface, R represents a gas constant (J/mol·K), and T represents a temperature (K) of the lubricant.)

According to the method for predicting an amount of hydrogen generated, the exposed area of the newly-generated surface is calculated using the sliding speed, and the sliding speed can realize a pure rolling condition or a rolling sliding condition in the rolling device between the two sliding members. Therefore, the hydrogen generation amount $\Delta H1$ due to sliding can reflect the amount of hydrogen generated in the rolling device. Therefore, when the exposed area of the newly-generated surface is the exposed area of the newly-generated surface in the rolling device, the amount of hydrogen generated in the rolling device can be predicted from the exposed area of the newly-generated surface and the above Formula (1). After the values of Ea and B in the Formula (1) are obtained, the hydrogen generation amount $\Delta H1$ can be calculated only by desktop calculation from the exposed area of the newly-generated surface, the temperature of the lubricant, and the Formula (1), so that the amount of hydrogen generated in the rolling device can be easily predicted.

The method for predicting an amount of hydrogen generated in a rolling device may further include a step of calculating a hydrogen generation amount $\Delta H2$ from the lubricant due to heat based on the following Formula (2).

$$\Delta H2 = C \times \exp(-Eb/RT)...\ (2)$$

(In the Formula (2), C represents a constant, Eb represents the activation energy (J/mol) of the lubricant in a reaction due to heat, R represents a gas constant (J/mol·K), and T represents a temperature (K) of the lubricant.)

According to the method for predicting an amount of hydrogen generated, the amount of hydrogen generated in the rolling device can be predicted from the exposed area of the newly-generated surface and the Formulas (1) and (2).

Therefore, the accuracy of the predicted hydrogen generation amount is further improved. This is for the following reason.

That is, when the temperature of the lubricant increases, the hydrogen generation amount also includes the amount of hydrogen generated from the lubricant due to heat. In particular, the higher the temperature of the lubricant, the higher the ratio of the amount of hydrogen generated from the lubricant due to heat to the hydrogen generation amount. Therefore, when not only the hydrogen generation amount $\Delta H1$ due to sliding but also the hydrogen generation amount $\Delta H2$ due to heat is considered when predicting the hydrogen generation amount, it is considered that the hydrogen generation amount calculated thereby approaches the actual hydrogen generation amount. Therefore, it is considered that the accuracy of the predicted hydrogen generation amount is further improved. After the values of Ea, Eb, B, and C in the Formulas (1) and (2) are obtained, the hydrogen generation amount $\Delta H1$ and the hydrogen generation amount $\Delta H2$ can be calculated only by desktop calculation from the exposed area of the newly-generated surface, the temperature of the lubricant, and the Formulas (1) and (2), so that the amount of hydrogen generated from the lubricant in the rolling device can be easily predicted.

In the method for predicting an amount of hydrogen generated, the metal contact ratio may be calculated by an impedance method.

In the method for predicting an amount of hydrogen generated, the exposed area of the newly-generated surface may be calculated based on the following Formula (X), and the width of the actual contact surface may be represented by the following Formula (Y).

$$\text{Exposed area of newly-generated surface} = D \times V \times t ... \quad (X)$$

(In the Formula (X), D represents a width (m) of the actual contact surface at an apparent contact surface between the sliding members, V represents a sliding speed (m/s), and t represents a contact time (s).)

$$\text{Width of actual contact surface} = 2 \times (\alpha \times Aa/\pi)^{1/2} ... \quad (Y)$$

(In the Formula (Y), Aa represents an area ($m^2$) of the apparent contact surface, and $\alpha$ represents the metal contact ratio.

In the method for predicting an amount of hydrogen generated, the metal contact ratio $\alpha$ may be represented by the following Formula (3).

$$\alpha = Ar/Aa ... \quad (3)$$

(In the Formula (3), Ar represents an area ($m^2$) of the actual contact surface.)

According to still another aspect of the present invention, there is provided a hydrogen generation test device capable of, while causing two sliding members having a lubricant interposed therebetween to slide against each other in a sealed container, measuring at least an amount of hydrogen generated from the lubricant, the hydrogen generation test device including: an impedance measuring device configured to measure an impedance between the two sliding members, in which the two sliding members are capable of changing a sliding speed between the two sliding members, the impedance is used to calculate a metal contact ratio between the two sliding members, and the metal contact ratio and the sliding speed are used to calculate an exposed area of a newly-generated surface due to direct contact between the two sliding members.

According to this hydrogen generation test device, it is possible to measure, while causing the two sliding members to slide against each other in the sealed container, the amount of hydrogen generated from the lubricant interposed between at least the two sliding members. On the other hand, the impedance between the two sliding members can be measured by an impedance measuring device. Therefore, the metal contact ratio between the two sliding members can be obtained using the impedance, and the exposed area of the newly-generated surface due to the direct contact between the two sliding members can be calculated using the sliding speed and the metal contact ratio.

In addition, the two sliding members can change the sliding speed between the two sliding members. Therefore, according to the hydrogen generation test device, a correlation between the exposed area of the newly-generated surface and the hydrogen generation amount can be obtained. Here, the exposed area of the newly-generated surface is calculated using the sliding speed, and the sliding speed can realize a pure rolling condition or a rolling sliding condition in the rolling device between the two sliding members. Therefore, the amount of hydrogen generated from the lubricant can reflect the amount of hydrogen generated in the rolling device. Therefore, when the exposed area of the newly-generated surface is the exposed area of the newly-generated surface in the rolling device, the amount of hydrogen generated in the rolling device can be predicted from the correlation.

The hydrogen generation test device may further include a heater disposed in the sealed container and configured to heat the lubricant; and a temperature detection device configured to detect a temperature of the lubricant.

The hydrogen generation test device may further include a gas port provided in the sealed container and configured to suck a gas containing hydrogen stored in a head space of the sealed container; and a hydrogen generation amount measuring device connected to the gas port via a tube and configured to measure an amount of hydrogen generated.

According to still another aspect of the present invention, there is provided a method for evaluating likelihood of white structure peeling occurring in a rolling device, the method including: a hydrogen generation amount measurement step in which, while two sliding members having a lubricant interposed therebetween are caused to slide against each other over a contact surface, an amount of hydrogen generated from the lubricant is measured; a threshold determination step in which a threshold of an amount of hydrogen generated in a case where white structure peeling is formed on the sliding members is determined based on the amount of hydrogen generated; a hydrogen generation amount prediction step in which an amount of hydrogen generated in the rolling device using the lubricant is predicted; and an evaluation step in which likelihood of white structure peeling occurring in the rolling device is evaluated by comparing the threshold of the amount of hydrogen generated with the amount of hydrogen generated that has been predicted in the hydrogen generation amount prediction step, in which the hydrogen generation amount prediction step is performed by the method for predicting an amount of hydrogen generated in a rolling device described above.

According to the method for evaluating the likelihood of white structure peeling occurring in a rolling device, the hydrogen generation amount prediction step is performed by the method for predicting the amount of hydrogen generated in the rolling device, and the amount of hydrogen generated from the lubricant in the rolling device can be predicted. Therefore, by comparing the threshold of the hydrogen generation amount with the hydrogen generation amount predicted in the hydrogen generation amount prediction step, the likelihood of white structure peeling occurring in the rolling device can be evaluated.

**Advantageous Effects of Invention**

According to the present invention, there are provided a method for predicting an amount of hydrogen generated in a rolling device capable of predicting an amount of hydrogen generated from a lubricant in a rolling device, a hydrogen generation test device used in the method, and a method for evaluating the likelihood of white structure peeling occurring in a rolling device.

**Brief Description of Drawings**

FIG. 1 is a schematic partial cross-sectional view illustrating an example of a hydrogen generation test device used in a method for predicting an amount of hydrogen generated in a rolling device according to one aspect of the present invention as viewed from a front direction.

FIG. 2 is a schematic partial cross-sectional view illustrating an example of the hydrogen generation test device used in the method for predicting an amount of hydrogen generated in a rolling device according to one aspect of the present invention from an upper surface direction.

FIG. 3 is a plan view schematically illustrating an apparent contact surface and a plurality of actual contact surfaces of two sliding members in FIG. 1.

FIG. 4 is a plan view schematically illustrating the apparent contact surface of the two sliding members in FIG. 1 and one actual contact surface equivalent to the plurality of actual contact surfaces.

FIG. 5 is a graph illustrating a relationship between a sliding speed and a hydrogen generation amount due to sliding.

FIG. 6 is a graph illustrating a relationship between a sliding speed and a metal contact ratio.

FIG. 7 is a graph illustrating a relationship between an exposed area of a newly-generated area and a hydrogen generation amount due to sliding.

FIG. 8 is a graph illustrating a relationship between a temperature of a lubricant and a hydrogen generation amount per exposed area of a newly-generated surface.

FIG. 9 is a graph illustrating a relationship between a temperature of a lubricant and a hydrogen generation amount due to heat.

**Description of Embodiments**

Hereinafter, embodiments of the present invention will be described in detail with reference to the drawings.

<Hydrogen Generation Test Device>

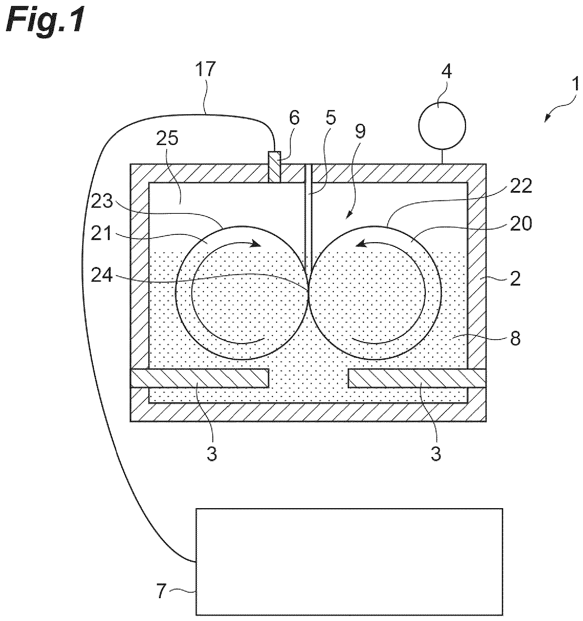

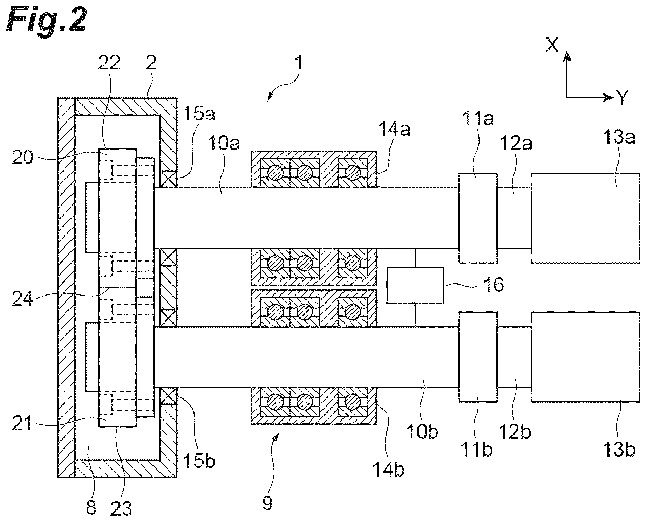

FIG. 1 is a schematic partial cross-sectional view illustrating an example of a hydrogen generation test device used in a method for predicting an amount of hydrogen generated in a rolling device according to one aspect of the present invention from a front direction, and FIG. 2 is a schematic partial cross-sectional view illustrating an example of the hydrogen generation test device used in the method for predicting an amount of hydrogen generated in a rolling device according to one aspect of the present invention from an upper surface direction.

As illustrated in FIGS. 1 and 2, a hydrogen generation test device 1 includes a two-cylinder testing mechanism 9 and a sealed container 2. The two-cylinder testing mechanism 9 includes a cylindrical metal sliding member 20 and a cylindrical metal sliding member 21. The metal constituting the sliding members 20 and 21 may be the same as a material used for the rolling device such as a rolling bearing or a sliding bearing, and as such metal, for example, steel is used. One sliding member 20 is fixed to one end of a shaft 10a rotatable about the central axis, and the other sliding member 21 is fixed to one end of a shaft 10b rotatable about the central axis. The sliding member 20 and sliding member 21 are accommodated inside the sealed container 2.

The shaft 10a is supported by the sealed container 2 via an oil seal 15a, and the other end of the shaft 10a is connected to a motor shaft 12a of a motor 13a via a coupling 11a. An intermediate portion between a portion of the shaft 10a supported by the oil seal 15a and a portion connected to the motor shaft 12a is supported by an inner ring of an intermediate support bearing 14a. An outer ring of the intermediate support bearing 14a is supported by a block (not illustrated). Similarly, the shaft 10b is supported by the sealed container 2 via an oil seal 15b, and the other end of the shaft 10b is connected to a motor shaft 12b of a motor 13b via a coupling 11b. An intermediate portion between a portion of the shaft 10b supported by the oil seal 15b and a portion connected to the motor shaft 12b is supported by an inner ring of an intermediate support bearing 14b. An outer ring of the intermediate support bearing 14b is supported by a block (not illustrated).

The shaft 10a is fixed on a linear guide (not illustrated) as an axial distance changing device, and can be moved by translational movement in a horizontal direction (X direction in FIG. 2). By applying a load to the shaft 10a that moves in the horizontal direction, an axial distance between the two shafts 10a and 10b can be changed, and a circumferential surface 22 of the sliding member 20 fixed to the shaft 10a and a circumferential surface 23 of the sliding member 21 fixed to the shaft 10b can be brought into contact with each other. In the present embodiment, although the shaft 10a is fixed on a linear guide (not illustrated) and can be moved by translational movement in the horizontal direction, instead of the shaft 10a, the shaft 10b may be fixed on the linear guide (not illustrated) as the axial distance changing device and can be moved by translational movement in the horizontal direction, and it is sufficient that at least one of the shaft 10a and the shaft 10b can be moved by translational movement in the horizontal direction.

The hydrogen generation test device 1 can apply a surface pressure to a contact surface 24 where the circumferential surface 22 of the sliding member 20 and the circumferential surface 23 of the sliding member 21 come into contact with each other by applying a load to the shaft 10a moving in the horizontal direction, and fixing the other shaft 10b. The oil seals 15a and 15b are oil seals that can be deformed to absorb the movement of the shafts 10a and 10b even when the shafts move in the horizontal direction. Therefore, even when at least one of the shaft 10a and the shaft 10b is moved by the translational movement in the horizontal direction, it is possible to maintain sealability in the sealed container 2.

The shaft 10a is rotatable about the axis by the connected motor 13a, and the shaft 10b is also rotatable about the axis by the connected motor 13b. Since the motors 13a and 13b are motors whose rotational speeds can be varied, a circumferential speed of the circumferential surface 22 of the sliding member 20 can be changed by changing the rotational speed of the motor 13a, and a circumferential speed of the circumferential surface 23 of the sliding member 21 can be changed by changing the rotational speed of the motor 13b. That is, in the sliding member 20 and the sliding member 21, a sliding speed (difference between the circumferential speed of the circumferential surface 22 of the sliding member 20 and the circumferential speed of the circumferential surface 23 of the sliding member 21) between the sliding member 20 and the sliding member 21 can be changed by the motors 13a and 13b. Therefore, when the circumferential speed of the circumferential surface 22 of the sliding member 20 and the circumferential speed of the circumferential surface 23 of the sliding member 21 are the same, the sliding member 20 and the sliding member 21 can come into contact with each other in a pure rolling state in which no sliding occurs, when the circumferential speed of the circumferential surface 22 of the sliding member 20 and the circumferential speed of the circumferential surface 23 of the sliding member 21 are different, the sliding member 20 and the sliding member 21 can come into contact with each other in a rolling sliding contact state, and when either one of the sliding member 20 and the sliding member 21 is stopped from rotating and the other is rotated, the sliding member 20 and the sliding member 21 can come into contact with each other in a pure sliding contact state.

A lubricating oil 8 is accommodated inside the sealed container 2 as a lubricant for lubricating the contact surface 24 between the sliding member 20 and the sliding member 21, the lubricating oil 8 is contained at a height sufficient to allow the contact surface 24, where the circumferential surface 22 of the sliding member 20 and the circumferential surface 23 of the sliding member 21 come into contact with each other, to be sufficiently immersed in the lubricating oil 8 from a bottom surface of the sealed container 2, and in an amount sufficient to secure a space (head space) 25 in the upper portion.

Therefore, inside the sealed container 2, the lower side including the contact surface 24 is filled with the lubricating oil 8, the upper side is secured with the head space 25 having no lubricating oil, and air is accommodated in the head space 25. Although air is accommodated in the sealed container 2, a type of gas in the sealed container 2 is not limited to air, and may be a gas other than air.

Inside the sealed container 2, a heater 3 for heating the lubricating oil 8 is disposed on the lower side, and a temperature detection device 5 for detecting the temperature of the lubricating oil 8 near the contact surface 24 is disposed. The temperature of the lubricating oil 8 is adjusted by the heater 3 and the temperature detection device 5. In the head space 25, a gas port 6 for sucking a gas containing hydrogen accumulated in the head space 25 and a pressure gauge 4 for detecting the pressure of the head space 25 are disposed. The gas port 6 is connected to a gas chromatograph 7 as a hydrogen generation amount measuring device via a tube 17. When it is desired to perform evaluation by setting the inside of the sealed container 2 to a vacuum state, a quadrupole mass spectrometer may be used as the hydrogen generation amount measuring device, and when it is desired to perform evaluation by using the quadrupole plasma mass spectrometer as the hydrogen generation amount measuring device, the inside of the sealed container 2 may be set to a vacuum state. As the temperature detection device 5, for example, a thermocouple is used.

The hydrogen generation test device 1 may further include an impedance measuring device 16 in order to insulate the intermediate support bearings 14a and 14b of the shafts 10a and 10b and the couplings 11a and 11b, connect the shafts 10a and 10b, and calculate a metal contact ratio $\alpha$ between the sliding member 20 and the sliding member 21 represented by the following Formula (3). As the impedance measuring device 16, for example, an LCR meter or the like can be used.

$$\alpha = Ar/Aa... \quad (3)$$

In the above Formula (3), Ar represents an area ($m^2$) of an actual contact surface between the two sliding members 20 and 21, and Aa represents an area ($m^2$) of the apparent contact surface 24.

The intermediate support bearings 14a and 14b and the couplings 11a and 11b are not necessarily required to be insulated.

For the hydrogen generation test device 1, a two-cylinder testing mechanism 9 that includes the shafts 10a and 10b that are capable of rotating about a central axis at a variable speed while fixing the cylindrical sliding member 20 and the cylindrical sliding member 21, respectively, and in which an axial distance between the shafts 10a and 10b is changeable, and the sliding member 20 and the sliding member 21 are in slidable contact with each other by changing the axial distance is used. Therefore, by changing the surface pressure of the contact surface 24 between the sliding member 20 and the sliding member 21, the circumferential speed due to the rotation of the sliding member 20, the circumferential speed due to the rotation of the sliding member 21, and the temperature of the lubricating oil 8, it is possible to change the area of the apparent contact surface 24 and the metal contact ratio.

In addition, in the hydrogen generation test device 1, each of the sliding member 20 and the sliding member 21 is rotatable at a variable speed about the central axis, and the circumferential speed due to the rotation of the sliding member 20 and the circumferential speed due to the rotation of the sliding member 21 can be arbitrarily controlled. Therefore, not only the pure sliding condition but also the same rolling sliding contact condition and pure rolling condition as those of the rolling device can be reproduced and evaluated.

Further, when the hydrogen generation test device 1 includes the impedance measuring device 16 that connects the shafts 10a and 10b, it is possible to calculate the metal contact ratio $\alpha$ between the sliding member 20 and the sliding member 21.

In addition, in the hydrogen generation test device 1, the sliding member 20 and the sliding member 21 are accommodated by using the sealed container 2, and air is accommodated inside the head space 25 of the sealed container 2. Therefore, the test atmosphere is not limited to a vacuum, and the test can be performed in an atmospheric environment.

<Method for Predicting Amount of Hydrogen Generated in Rolling Device>

(First Embodiment)

Next, a first embodiment of the method for predicting an amount of hydrogen generated in a rolling device of the present invention will be described.

First, using the hydrogen generation test device 1, while the two sliding members 20 and 21 having the lubricating oil 8 interposed therebetween are caused to slide against each other over the contact surface 24, an exposed area of a newly-generated surface due to direct contact between the two sliding members 20 and 21 is calculated after t (s) elapses from the start of sliding of the two sliding members 20 and 21, and an amount of hydrogen generated from the lubricating oil 8 is measured.

At this time, the surface pressure (GPa), the sliding speed V (m/s), the temperature T (K) of the lubricating oil 8, and the pull-in speed v (m/s) of the lubricating oil 8 at the contact surface 24 where the circumferential surface 22 of the sliding member 20 and the circumferential surface 23 of the sliding member 21 come into contact with each other are kept constant.

Here, the sliding speed V is the sliding speed between the two sliding members 20 and 21, and is the difference between the circumferential speed at the circumferential surface 22 of the sliding member 20 and the circumferential speed at the circumferential surface 23 of the sliding member 21. The pull-in speed v is an average of the circumferential speed at the circumferential surface 22 of the sliding member 20 and the circumferential speed at the circumferential surface 23 of the sliding member 21. For example, when the circumferential speed at the circumferential surface 22 of the sliding member 20 is 1.5 m/s and the circumferential speed at the circumferential surface 23 of the sliding member 21 is 0.5 m/s, the sliding speed V is (1.5 - 0.5) = 1 m/s, and the pull-in speed is (1.5 + 0.5)/2 = 1 m/s.

At this time, an exposed area S ($m^2$) of the newly-generated surface is calculated by using the metal contact ratio $\alpha$ and the sliding speed V between the two sliding members 20 and 21. Specifically, the exposed area S of the newly-generated surface is calculated based on the following Formula (X).

$$\text{Exposed area S of newly-generated surface} = D \times V \times t... \quad (X)$$

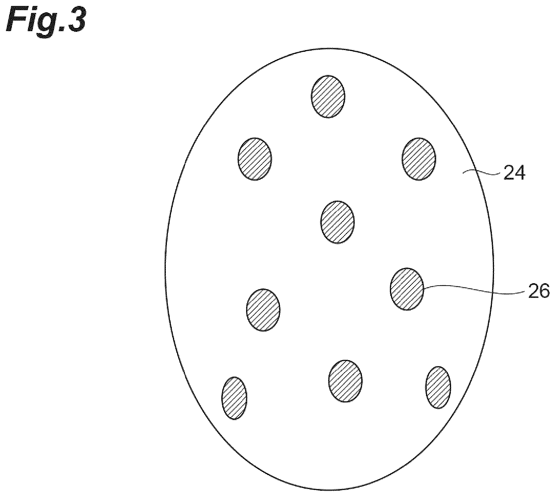

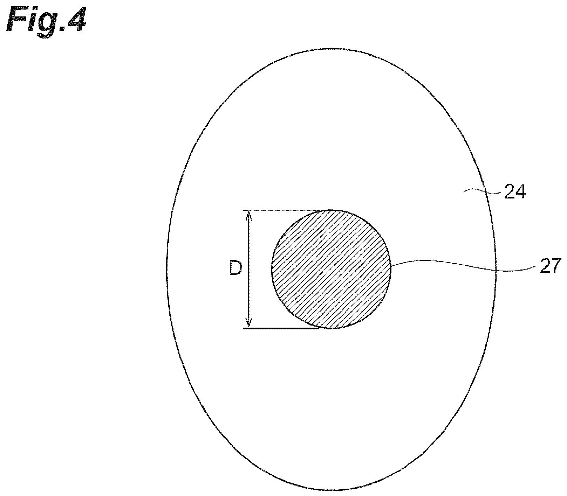

In the above Formula (X), D represents a width (m) of the actual contact surface at the apparent contact surface 24 between the two sliding members 20 and 21, V represents the sliding speed (m/s), t represents a contact time (s), and is a time during which the circumferential surface 22 of the sliding member 20 and the circumferential surface 23 of the sliding member 21 are in contact with each other. Here, as illustrated in FIG. 3, there are usually a plurality of actual contact surfaces 26 at the apparent contact surface 24, but here, as illustrated in FIG. 4, one circular actual contact surface 27 having an area equal to a total area of the plurality of actual contact surfaces 26 is considered, and the diameter of the actual contact surface 27 is defined as the width D (m) of the actual contact surface. D is specifically represented by the following Formula (Y).

$$D = 2 \times (\alpha \times Aa/\pi)^{1/2}... \quad (Y)$$

In the above Formula (Y), Aa represents an area ($m^2$) of the apparent contact surface 24, and $\alpha$ represents a metal contact ratio represented by the following Formula (3).

$$\alpha = Ar/Aa... \quad (3)$$

In the above Formula (3), Ar represents an area ($m^2$) of the actual contact surface 27.

Here, the area Aa of the apparent contact surface 24 is obtained by, for example, Hertz contact theory.

The metal contact ratio $\alpha$ is calculated by, for example, an impedance method. In the impedance method, an AC voltage is applied between the sliding member 20 and the sliding member 21 by the impedance measuring device 16 of the hydrogen generation test device 1, an impedance Z and a phase angle $\theta$ in an electric circuit formed by the sliding member 20, the lubricating oil 8, and the sliding member 21 are measured and output, and the metal contact ratio $\alpha$ is calculated based on the impedance Z and the phase angle $\theta$.

On the other hand, the amount of hydrogen generated from the lubricating oil 8 is measured by collecting a gas containing hydrogen from the head space 25 in the sealed container 2 to the gas chromatograph 7 through the gas port 6 and the tube 17 and performing analysis by the gas chromatograph 7. The hydrogen generation amount is represented by a hydrogen concentration (ppm) in the gas.

At this time, it is preferable that the hydrogen generation amount H is repeatedly measured under the same conditions until the hydrogen generation amount is stabilized, and a stable hydrogen generation amount is used as a measured value. Dissolved moisture exists in the lubricating oil 8, and although there is likelihood that the moisture is decomposed to generate hydrogen, after the hydrogen generation amount is repeatedly measured under the same conditions, the hydrogen generation amount due to the dissolved moisture can be reduced. Therefore, the prediction accuracy of the predicted hydrogen generation amount is further improved.

Next, the exposed area S of the newly-generated surface is changed by changing the metal contact ratio $\alpha$, the sliding speed V, and the contact time t between the two sliding members 20 and 21, and hydrogen is generated in the hydrogen generation test device 1. Thereafter, this operation is repeated.

As described above, a correlation between the exposed area S of the newly-generated surface and the hydrogen generation amount H is obtained. However, while this correlation is obtained, for example, the temperature T (K) of the lubricating oil 8 and the pull-in speed v of the lubricating oil 8 are kept constant. When the correlation is obtained, the contact time t does not necessarily need to be changed and may be constant.

According to the method for predicting an amount of hydrogen generated, the exposed area S of the newly-generated surface is calculated by using the sliding speed V, and the sliding speed V can realize a pure rolling condition or a rolling sliding condition in the rolling device between the two sliding members 20 and 21. Therefore, the amount of hydrogen generated from the lubricating oil 8 can reflect the amount of hydrogen generated from the lubricating oil in the rolling device. Therefore, when the exposed area S of the newly-generated surface is the exposed area of the newly-generated surface in the rolling device using the lubricating oil, the amount of hydrogen generated from the lubricating oil in the rolling device can be predicted from the exposed area S of the newly-generated surface and the correlation.

Here, an exposed area S1 of a newly-generated surface in the rolling device using a lubricating oil can be obtained in the same manner as the exposed area S of the newly-generated surface in the hydrogen generation test device 1. Specifically, the exposed area S1 of the newly-generated surface is represented by the following Formula (X1).

$$\text{Exposed area S1 of newly-generated surface} = D1 \times V1 \times t1... \text{ (X1)}$$

In the above Formula (X1), D1 represents a width (m) of an actual contact surface between the sliding members in the rolling device, V1 represents a sliding speed (m/s), and t1 represents a contact time (s). Here, although there is usually a plurality of actual contact surfaces in the contact surface, here, one circular newly-generated surface having an area equal to a total area of the plurality of actual contact surfaces is considered, and the diameter D1 (m) of the actual contact surface is defined as a width of the newly-generated surface. D1 is specifically represented by the following Formula (Y1).

$$D1 = 2 \times (\alpha 1 \times Aa1/\pi)^{1/2}... \text{ (Y1)}$$

In the above Formula (Y1), Aa1 represents an area (m$^2$) of the apparent contact surface, and $\alpha 1$ represents a metal contact ratio represented by the following Formula (3A).

$$\alpha = Ar1/Aa1... \text{ (3A)}$$

In the above Formula (3A), Ar1 represents an area (m$^2$) of the actual contact surface.

Here, the area Aa1 of the apparent contact surface can be obtained in the same manner as the area of the apparent contact surface 24 of the two sliding members 20 and 21 of the hydrogen generation test device 1. Although Aa1 represents the area of the apparent contact surface, since the rolling device has a plurality of rolling elements and each rolling element is in contact with the inner ring and the outer ring, the area of the contact surface is calculated based on the number of rolling elements and the number of surfaces on which the rolling elements are in contact with the inner ring and the outer ring.

A metal contact ratio $\alpha 1$ is calculated by, for example, an impedance method. In the impedance method, when the rolling device includes an inner ring, an outer ring, and a plurality of rolling elements slidably provided on an outer circumference of the inner ring and an inner circumference of the outer ring between the inner ring and the outer ring, an AC voltage is applied between the inner ring and the outer ring by an impedance measuring device electrically connected to the inner ring and the outer ring, the impedance Z and the phase angle $\theta$ in the electric circuit formed by the inner ring, the plurality of rolling elements, and the outer ring are measured and output, and the metal contact ratio $\alpha$ is calculated based on the impedance Z and the phase angle $\theta$.

(Second Embodiment)

Next, a second embodiment of the method for predicting an amount of hydrogen generated in a rolling device of the present invention will be described.

In the present embodiment, using the hydrogen generation test device 1, while the two sliding members 20 and 21 having the lubricant interposed therebetween are caused to slide against each other over the contact surface 24, the exposed area of the newly-generated surface due to the direct contact between the two sliding members 20 and 21 is calculated after t (s) elapses from the start of sliding of the two sliding members 20 and 21 (first step).

The exposed area of the newly-generated surface is calculated by using the metal contact ratio $\alpha$ and the sliding speed V between the two sliding members 20 and 21. Specifically, the exposed area S of the newly-generated surface can be measured in the same manner as in the first embodiment.

Next, a hydrogen generation amount ΔH1 due to the sliding from the apparent contact surface 24 is calculated based on the exposed area S of the newly-generated surface and the following Formula (1) (second step).

$$\Delta H1/S = B \times \exp(\text{-Ea/RT})...\ (1)$$

In the above Formula (1), B represents a constant, S represents an exposed area ($m^2$) of the newly-generated surface, Ea represents activation energy (J/mol) of the lubricating oil 8 in a reaction with the newly-generated surface, R represents a gas constant (J/mol·K), and T represents a temperature (K) of the lubricating oil 8.

The above B and Ea can be obtained, for example, by the following method.

That is, first, the hydrogen generation amount ΔH1 and the exposed area S of the newly-generated surface when the temperature T of the lubricating oil 8 is T1 are obtained. At this time, in the hydrogen generation test device 1, the two sliding members 20 and 21 are rotated while sliding against each other over the contact surface 24 with the lubricant interposed between the two sliding members 20 and 21.

Next, in the hydrogen generation test device 1, the temperature T of the lubricating oil 8 is changed to T2 by the heater 3 and the temperature detection device 5, and the hydrogen generation amount ΔH1 and the exposed area S of the newly-generated surface at that time are obtained. Also at this time, in the hydrogen generation test device 1, the two sliding members 20 and 21 are rotated while sliding against each other over the contact surface 24 with the lubricant interposed between the two sliding members 20 and 21.

Then, in a graph where a vertical axis is ln(ΔH1/S) and a horizontal axis is 1/T, ln(ΔH1/S) when T is T1 and ln(ΔH1/S) when T is T2 are plotted, and a formula of a straight line connecting the plotted points is obtained. This formula is represented by the following Formula (1A).

$$\ln(\Delta H1/S) = a \times (1/T) + b...\ (1A)$$

Here, the straight line is an approximate straight line obtained by a least squares method or the like.

On the other hand, from the above Formula (1), ln(ΔH1/S) is represented by the following Formula (1B).

$$\ln(\Delta H1/S) = (\text{-Ea/R}) \times (1/T) + \ln B...\ (1B)$$

The following relationship is established from the above Formulas (1A) and (1B).

$$a = \text{-Ea/R}$$

$$b = \ln B$$

Therefore, B and Ea are obtained as follows.

$$B = \exp(b)$$

$$Ea = \text{-}a \times R$$

In addition, the above Formula (1) was found through experiments conducted by the present inventors on the assumption that the hydrogen generation amount per exposed area of the newly-generated surface follows Arrhenius' law.

In the above method, B and Ea are obtained based on two temperatures T and their corresponding ln(ΔH1/S), but it is preferable to obtain B and Ea based on three or more temperatures and their corresponding ln(ΔH/S). In this case, the reliability of the values of B and Ea is further improved, and the reliability of the above Formula (1) is also further improved. As a result, reliability of the hydrogen generation amount due to the sliding from the apparent contact surface 24, which is predicted based on the above Formula (1), is further improved.

According to the method for predicting an amount of hydrogen generated, the exposed area of the newly-generated surface is calculated using the sliding speed, and the sliding speed can realize a pure rolling condition or a rolling sliding condition in the rolling device between the two sliding members 20 and 21. Therefore, the hydrogen generation amount ΔH1 due to sliding can reflect the amount of hydrogen generated from the lubricant in the rolling device. Therefore, when the exposed area of the newly-generated surface is the exposed area of the newly-generated surface in the rolling device using the lubricant, the amount of hydrogen generated from the lubricant in the rolling device can be predicted from the exposed area S of the newly-generated surface and the above Formula (1). After the values of Ea and B in Formula (1) are obtained, the hydrogen generation amount $\Delta H1$ can be calculated only by desktop calculation from the exposed area of the newly-generated surface, the temperature of the lubricating oil 8, and Formula (1) without using the hydrogen generation test device 1, so that the amount of hydrogen generated from the lubricant in the rolling device can be easily predicted.

The method for predicting an amount of hydrogen generated of the present embodiment may further include a step of calculating a hydrogen generation amount $\Delta H2$ from the lubricant due to heat based on the following Formula (2), and the amount of hydrogen generated from the lubricant in the rolling device may be predicted based on the hydrogen generation amount $\Delta H1$ and the hydrogen generation amount $\Delta H2$.

$$\Delta H2 = C \times \exp(-Eb/RT)... \quad (2)$$

In the above Formula (2), C represents a constant, Eb represents activation energy (J/mol) of the lubricating oil 8 in a reaction due to heat, R represents a gas constant (J/mol·K), and T represents a temperature (K) of the lubricating oil 8.

C and Eb above can be obtained by, for example, the following method.

That is, first, the hydrogen generation amount $\Delta H2$ when the temperature T of the lubricating oil 8 is T1 is obtained. At this time, the two sliding members 20 and 21 are rotated in a non-contact state in the hydrogen generation test device 1 in order to stir the lubricating oil 8 to make the temperature thereof uniform while preventing the exposure of the newly-generated surface due to the contact between the two sliding members 20 and 21.

Next, in the hydrogen generation test device 1, the temperature T of the lubricating oil 8 is changed to T2 by the heater 3 and the temperature detection device 5, and the hydrogen generation amount $\Delta H2$ at that time is obtained. Also at this time, in the hydrogen generation test device 1, the two sliding members 20 and 21 are rotated in a non-contact state.

Then, in a graph where a vertical axis is $\ln(\Delta H2)$ and a horizontal axis is $1/T$, $\ln(\Delta H2)$ when T is T1 and $\ln(\Delta H2)$ when T is T2 are plotted, and a formula of a straight line connecting the plotted points is obtained. This formula is represented by the following Formula (2A).

$$\ln(\Delta H2) = c(1/T) + d... \quad (2A)$$

Here, the straight line is an approximate straight line obtained by a least squares method or the like.

On the other hand, from the above Formula (2), $\ln(\Delta H2)$ is represented by the following Formula (2B).

$$\ln(\Delta H2) = -Eb/R(1/T) + \ln C... \quad (2B)$$

The following relationship is established from the above Formulas (2A) and (2B).

$$c = -Eb/R$$

d=lnC

Therefore, C and Eb are obtained as follows.

$$C = \exp(d)$$

$$Eb = -c \times R$$

It is to be noted that the above Formula (2) was found through experiments conducted by the present inventors on the assumption that the hydrogen generation amount $\Delta H2$ from the lubricating oil 8 due to heat follows Arrhenius' law.

In the above method, C and Eb are obtained based on two temperatures T and their corresponding $\ln(\Delta H2)$, but it is preferable to obtain C and Eb based on three or more temperatures and their corresponding $\ln(\Delta H2)$. In this case, reliability of the values of C and Eb is further improved, and reliability of the above Formula (2) is also further improved. As a result, reliability of the amount of hydrogen generated from the lubricant due to heat, which is predicted based on the above Formula (2), is further improved.

According to the method for predicting an amount of hydrogen generated, the amount of hydrogen generated from the lubricating oil 8 in the rolling device can be predicted from the exposed area of the newly-generated surface and the Formulas (1) and (2). Therefore, the accuracy of the predicted hydrogen generation amount is further improved. This is for the following reasons.

That is, when the temperature of the lubricating oil 8 increases, the hydrogen generation amount also includes the amount of hydrogen generated from the lubricating oil 8 due to heat. In particular, the higher the temperature of the lubricating oil 8, the higher the ratio of the amount of hydrogen generated from the lubricating oil 8 due to heat to the hydrogen generation amount. Therefore, when not only the hydrogen generation amount $\Delta H1$ due to sliding but also the hydrogen generation amount $\Delta H2$ due to heat are considered when predicting the hydrogen generation amount, it is considered that the hydrogen generation amount calculated thereby approaches the actual hydrogen generation amount. Therefore, it is considered that the accuracy of the predicted hydrogen generation amount is further improved.

After the values of Ea, Eb, B, and C in the Formulas (1) and (2) are obtained, the hydrogen generation amount $\Delta H1$ and the hydrogen generation amount $\Delta H2$ can be calculated only by desktop calculation from the exposed area of the newly-generated surface, the temperature of the lubricating oil 8, and the Formulas (1) and (2) without using the hydrogen generation test device 1, and thus the amount of hydrogen generated from the lubricant in the rolling device can be easily predicted.

<Method for Evaluating Likelihood of White structure Peeling Occurring in Rolling Device>

Next, a method for evaluating the likelihood of white structure peeling occurring in a rolling device of the present embodiment will be described.

First, using the hydrogen generation test device 1, the amount of hydrogen generated from the lubricating oil 8 is measured while the two sliding members 20 and 21 having the lubricating oil 8 interposed therebetween are caused to slide against each other over the contact surface 24 (hydrogen generation amount measurement step).

At this time, the measurement of the hydrogen generation amount may be performed in the same manner as the measurement of the hydrogen generation amount in the first embodiment of the method for predicting an amount of hydrogen generated in a rolling device.

On the other hand, whether white structure peeling is formed on the two sliding members 20 and 21 is observed.

Next, the test conditions are changed, the hydrogen generation amount is measured, and whether white structure peeling is formed on the two sliding members 20 and 21 is observed. Thereafter, this operation is repeated.

Then, when white structure peeling is formed in the two sliding members 20 and 21, the hydrogen generation amount at that time is determined as a threshold of the hydrogen generation amount in a case where white structure peeling is formed in the two sliding members 20 and 21 (threshold determination step).

Next, the amount of hydrogen generated in the rolling device using the lubricating oil 8 is predicted (hydrogen generation amount prediction step).

At this time, the hydrogen generation amount prediction step is performed by the method for predicting an amount of hydrogen generated in a rolling device of the first embodiment or the second embodiment described above.

Next, by comparing the threshold of the hydrogen generation amount with the hydrogen generation amount predicted in the hydrogen generation amount prediction step, the likelihood of white structure peeling occurring in the rolling device is evaluated (evaluation step).

According to the method for evaluating likelihood of white structure peeling occurring in a rolling device, the hydrogen generation amount prediction step is performed by the method for predicting an amount of hydrogen generated in a rolling device of the first embodiment or the second embodiment described above, and the amount of hydrogen generated from the lubricant in the rolling device can be predicted. Therefore, by comparing the threshold of the hydrogen generation amount with the hydrogen generation amount predicted in the hydrogen generation amount prediction step, the likelihood of white structure peeling occurring in the rolling device can be evaluated.

Hereinafter, results of the experiments performed in the first embodiment and the second embodiment will be described.

<Experimental Example 1: Evaluation of Relationship between Sliding Speed and Hydrogen Generation Amount>

(Hydrogen Generation Test Device 1A)

In the hydrogen generation test device 1, a hydrogen generation test device 1A was prepared in which an oil of VG32 was used as the lubricating oil 8, the temperature of the lubricating oil 8 was set to 70°C, and the surface roughness Ra of the circumferential surfaces 22 and 23 of the cylindrical steel sliding members 20 and 21 (outer diameter: 6 cm, thickness: 1.6 cm) was set to 0.02 µm.

(Hydrogen Generation Test Device 1B)

In the hydrogen generation test device 1, a hydrogen generation test device 1B was prepared in which an oil of VG32 was used as the lubricating oil 8, a temperature of the lubricating oil 8 was set to 70°C, and a surface roughness Ra of the circumferential surfaces of the cylindrical steel sliding members 20 and 21 (outer diameter: 6 cm, thickness: 1.6 cm) was set to 0.07 μm.

(Hydrogen Generation Test Device 1C)

In the hydrogen generation test device 1, a hydrogen generation test device 1C was prepared in which an oil of VG32 was used as the lubricating oil 8, the temperature of the lubricating oil 8 was set to 90°C, and the surface roughness Ra of the circumferential surfaces of the cylindrical steel sliding members 20 and 21 (outer diameter: 6 cm, thickness: 1.6 cm) was set to 0.07 μm.

(Hydrogen Generation Test Device 1D)

In the hydrogen generation test device 1, a hydrogen generation test device 1D was prepared in which an oil of VG32 was used as the lubricating oil 8, a temperature of the lubricating oil 8 was set to 80°C, and a surface roughness Ra of the circumferential surfaces of the cylindrical steel sliding members 20 and 21 (outer diameter: 6 cm, thickness: 1.6 cm) was set to 0.07 μm.

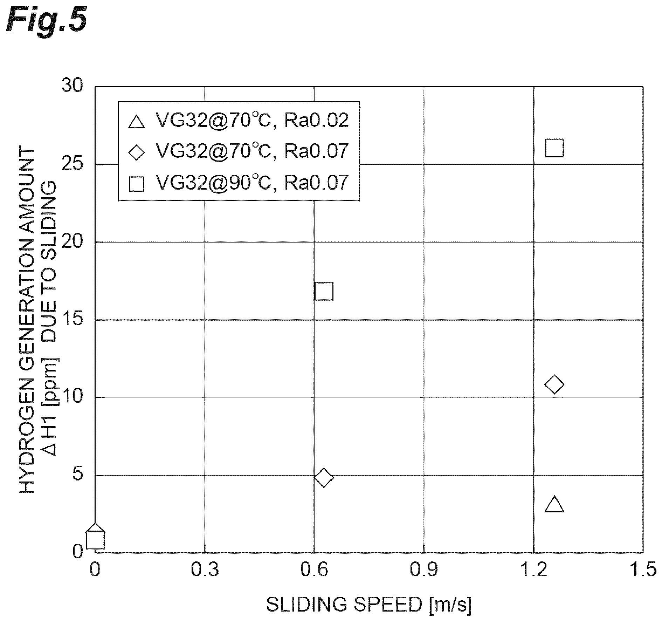

Then, for the hydrogen generation test device 1A, the hydrogen generation amount ΔH1 (ppm) due to sliding when the sliding speed was 1.26 m/s was measured, for the hydrogen generation test device 1B, the hydrogen generation amount ΔH1 (ppm) due to sliding when the sliding speed was 0 m/s, 0.63 m/s, and 1.26 m/s was measured, and for the hydrogen generation test device 1C, the hydrogen generation amount ΔH1 (ppm) due to sliding when the sliding speed was 0 m/s, 0.63 m/s, and 1.26 m/s was measured and plotted. The results are illustrated in FIG. 5.

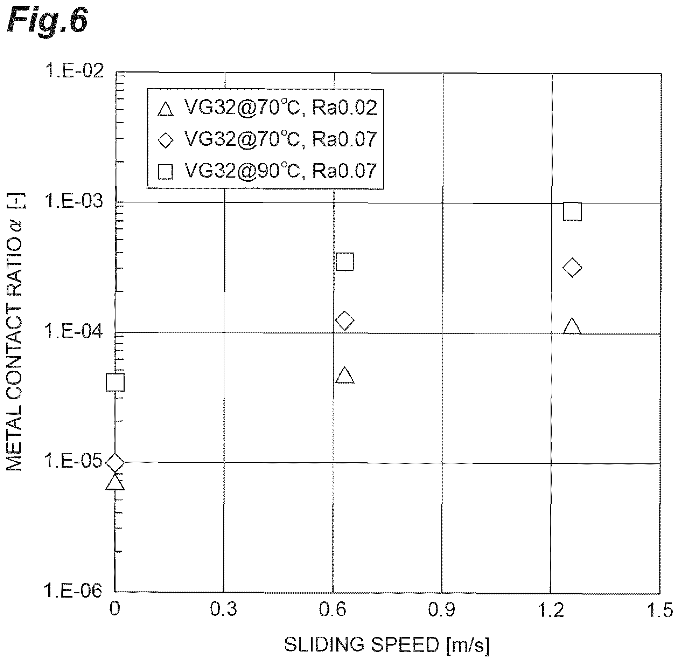

In addition, for the hydrogen generation test devices 1A, 1B, and 1C, the metal contact ratio $\alpha$ when the sliding speed was 0 m/s, 0.63 m/s, and 1.26 m/s was obtained and plotted. The results are illustrated in FIG. 6. At this time, the metal contact ratio $\alpha$ was obtained by the impedance method. Specifically, an AC voltage (frequency: 100 kHz) of 0.2 V was applied between the sliding member 20 and the sliding member 21 by the impedance measuring device 16 of the hydrogen generation test device 1, and the impedance Z and the phase angle $\theta$ in the electric circuit formed by the sliding member 20, the lubricating oil 8, and the sliding member 21 were measured and output, and the metal contact ratio $\alpha$ was calculated based on the impedance Z and the phase angle $\theta$.

Further, in the hydrogen generation test device 1A, 1B, and 1C, D was calculated from the metal contact ratio $\alpha$ under the sliding speed condition where the hydrogen generation amount ΔH1 (ppm) was measured and the area Aa of the apparent contact surface based on the Formula (Y). At this time, the apparent contact surface was an ellipse, and the area Aa of the apparent contact surface was obtained from the following formula from the Hertz contact theory.

$$Aa = \pi \times a \times b ... \; (4)$$

Here, a and b are a major axis radius (m) and a minor axis radius (m) of the ellipse of the contact surface, respectively, and were obtained from the following.

$$a = \mu(3 \times Q \times (\theta1 + \theta2)/(2 \times \Sigma\rho))^{1/3} ... \; (5)$$

$$b = \nu(3 \times Q \times (\theta1 + \theta2)/(2 \times \Sigma\rho))^{1/3} ... \; (6)$$

$$\theta1 = (1 - \lambda1^2)/\varepsilon1 ... \; (7)$$

$$\theta2 = (1 - \lambda2^2)/\varepsilon2 ... \; (8)$$

$$\Sigma\rho = 1/RX + 1/RY ... \; (9)$$

$$1/RX = 1/r1X + 1/r2X... \quad (10)$$

$$1/RY = 1/r1Y + 1/r2Y... \quad (11)$$

$$\mu = (2 \times \kappa^2 \times E/\pi)^{1/3}... \quad (12)$$

$$\nu = (2 \times E/(\pi \times \kappa))^{1/3}... \quad (13)$$

$$\kappa = 1.0339 \times k^{0.636}... \quad (14)$$

$$E = 1.0003 + 0.5968/k... \quad (15)$$

$$k = RX/RY... \quad (16)$$

In the above formula, Q is a load (N) between the sliding member 20 and the sliding member 21, $\lambda 1$ and $\lambda 2$ are Poisson's ratios of the sliding member 20 and the sliding member 21, respectively, and $\varepsilon 1$ and $\varepsilon 2$ are Young's moduli (N/m$^2$) of the sliding member 20 and the sliding member 21, respectively. Further, r1X is a radius (m) when the outer diameter of the sliding member 20 is a diameter, r1Y is a radius of curvature (m) of an end surface (circumferential surface 22) of the sliding member 20, r2X is a radius (m) when the outer diameter of the sliding member 21 is a diameter, and r2Y is a radius of curvature (m) of an end surface (circumferential surface 23) of the sliding member 21.

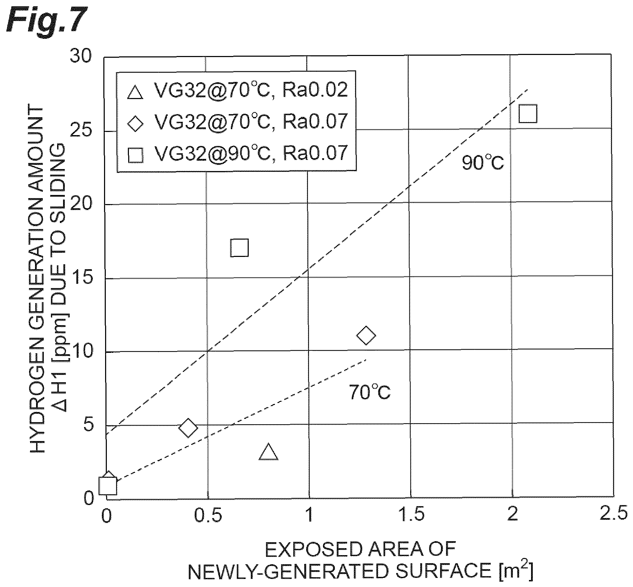

Then, the exposed area S of the newly-generated surface was obtained from D and V, the contact time t, and the above Formula (X). At this time, the contact time t was set to 20 hours. Then, the hydrogen generation amount $\Delta H1$ (ppm) due to sliding with respect to the exposed area of the newly-generated surface was plotted. The results are illustrated in FIG. 7.

In FIGS. 5 to 7, the results of the hydrogen generation test devices 1A, 1B, and 1C were set to "□", "□", and "□", respectively. The surface pressure in each of the hydrogen generation test devices 1A, 1B, and 1C was set to 2.3 GPa, the pull-in speed was set to 1.6 m/s, and the contact time (test time) was set to 20 hours.

From the results illustrated in FIG. 7, it has been found that there is a good correlation between the exposed area S of the newly-generated surface and the hydrogen generation amount $\Delta H1$ due to sliding regardless of whether the temperature of the lubricating oil is 70°C or 90°C.

<Experimental Example 2: Evaluation of Relationship between Temperature of Lubricating Oil and Hydrogen Generation Amount Per Exposed Area of Newly-Generated Surface>

From the results illustrated in FIG. 7, the hydrogen generation amount ($\Delta H1/S$) per exposed area of the newly-generated surface was obtained.

In addition, in order to investigate an influence of temperature in detail, an additional test was performed using the hydrogen generation test device 1D in which the temperature of the lubricating oil 8 was set to 80°C. In this additional test, the hydrogen generation amount $\Delta H1$ (ppm) due to sliding when the sliding speed was 1.26 m/s was measured to obtain the metal contact ratio $\alpha$. The surface pressure was set to 2.3 GPa, the pull-in speed was set to 1.6 m/s, and the contact time (test time) was set to 20 hours. Then, the hydrogen generation amount ($\Delta H1/S$) per exposed area of the newly-generated surface was obtained in the same manner as in the test using the hydrogen generation test devices 1A, 1B, and 1C.

In addition, additional tests were performed using the hydrogen generation test devices 1B and 1C in which the temperature of the lubricating oil 8 was set to 70°C and 90°C. In this additional test, the hydrogen generation amount $\Delta H1$ (ppm) due to sliding when the sliding speed was 1.26 m/s was measured to obtain the metal contact ratio $\alpha$. The surface pressure was set to 2.3 GPa, the pull-in speed was set to 1.6 m/s, and the contact time (test time) was set to 20 hours. Then, similarly, the hydrogen generation amount ($\Delta H1/S$) per exposed area of the newly-generated surface was obtained.

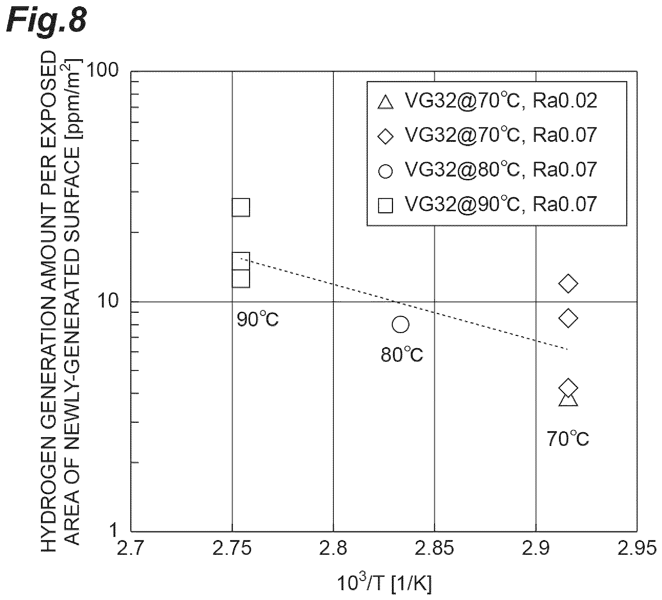

Then, the hydrogen generation amount ($\Delta H1/S$) per exposed area of the newly-generated surface was plotted with respect to $10^3/T$. The results are illustrated in FIG. 8. Note that the vertical axis was expressed in logarithmic scale.

From the results illustrated in FIG. 8, it is suggested that there is a good correlation between the reciprocal of the temperature and the logarithm of the hydrogen generation amount per exposed area of the newly-generated surface, and the hydrogen generation due to sliding follows Arrhenius' law.

<Experimental Example 3: Evaluation of Relationship between Temperature of Lubricating Oil and Hydrogen Generation Amount Due to Heat>

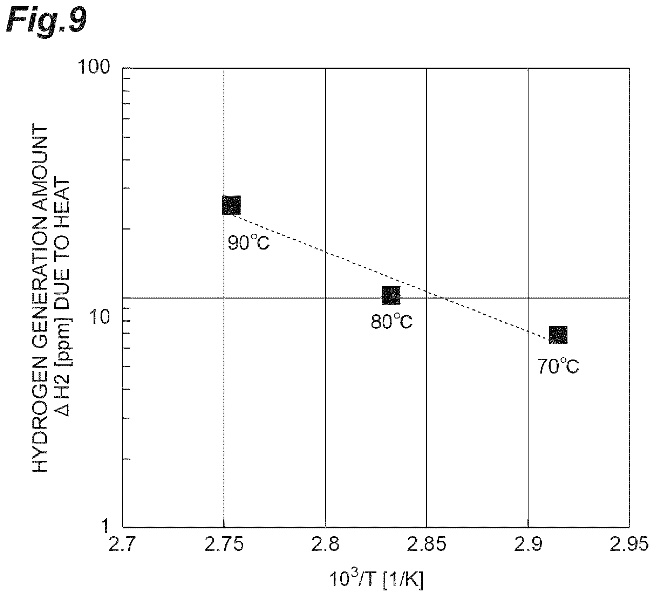

In the hydrogen generation test devices 1B, 1C, and 1D, the hydrogen generation amount when the sliding speed was 0 m/s was measured as the hydrogen generation amount ($\Delta$H2) due to heat, and plotted. The results are indicated in FIG. 9. Note that the vertical axis was expressed in logarithmic scale.

From the results illustrated in FIG. 9, it is suggested that there is a good correlation between the reciprocal of temperature and the logarithm of the amount of hydrogen generated due to heat, and the hydrogen generation due to heat follows Arrhenius' law.

In Experimental Examples 1 to 3, the result of only the VG32 as the lubricating oil is indicated, but even when an oil type different from the VG32 is used, it is considered that a good correlation can be obtained between various factors (sliding speed and exposed area of the newly-generated surface) and the hydrogen generation amount, between the reciprocal of the temperature of the lubricating oil and the hydrogen generation amount per exposed area of the newly-generated surface, and between the reciprocal of the temperature of the lubricating oil and the hydrogen generation amount due to heat. This is because the exposed area of the newly-generated surface and the temperature are considered to be factors of hydrogen generation regardless of the oil type.

In the hydrogen generation test device 1 according to the present embodiment, although the lubricating oil 8 is used as the lubricant, grease or a solid lubricant may be used instead of the lubricating oil. When the grease or solid lubricant is used, the grease or solid lubricant may be supplied to the contact surface 24 using, for example, a grease gun or the like in order to immerse the contact surface 24 where the circumferential surface 22 of the sliding member 20 and the circumferential surface 23 of the sliding member 21 come into contact with each other in the grease or solid lubricant.

**Reference Signs List**

1 Hydrogen generation test device
2 Sealed container
3 Heater
4 Pressure gauge
5 Temperature detection unit
6 Gas port
7 Gas chromatograph
8 Lubricating oil
9 Two-cylinder testing mechanism
10a, 10b Shaft
11a, 11b Coupling
12a, 12b Motor shaft
13a, 13b Motor
14a, 14b Intermediate support bearing
15a, 15b Oil seal
16 Impedance measuring device
17 Tube
20, 21 Sliding member
22, 23 Circumferential surface
24 Apparent contact surface
25 Head space
26, 27 Actual contact surface

**Claims**

1. A method for predicting an amount of hydrogen generated in a rolling device, the method comprising:

a step in which, while two sliding members are caused to slide against each other over a contact surface with a lubricant interposed between the two sliding members, an exposed area of a newly-generated surface due to direct contact between the two sliding members is calculated, an amount of hydrogen generated from the lubricant is measured, and a correlation between the exposed area of the newly-generated surface and the amount of hydrogen generated is obtained, wherein
the exposed area of the newly-generated surface is calculated using a metal contact ratio and a sliding speed of the two sliding members.

2. A method for predicting an amount of hydrogen generated in a rolling device, the method comprising:

a first step in which, while two sliding members are caused to slide against each other over a contact surface with a lubricant interposed between the two sliding members, an exposed area of a newly-generated surface due to direct contact between the two sliding members is calculated; and
a second step in which a hydrogen generation amount $\Delta H1$ due to sliding is calculated based on the exposed area of the newly-generated surface and the following Formula (1), wherein
the exposed area of the newly-generated surface is calculated using a metal contact ratio and a sliding speed of the two sliding members,

$$\Delta H1/S = B \times \exp(-Ea/RT)... \quad (1)$$

wherein B represents a constant, S represents an exposed area ($m^2$) of the newly-generated surface, Ea represents activation energy (J/mol) of the lubricant in a reaction with the newly-generated surface, R represents a gas constant (J/mol·K), and T represents a temperature (K) of the lubricant.

3. The method for predicting an amount of hydrogen generated in a rolling device according to claim 2, further comprising:

a step of calculating a hydrogen generation amount $\Delta H2$ due to heat based on the following Formula (2),

$$\Delta H2 = C \times \exp(-Eb/RT)... \quad (2)$$

wherein C represents a constant, Eb represents activation energy (J/mol) of the lubricant in a reaction due to heat, R represents a gas constant (J/mol·K), and T represents a temperature (K) of the lubricant.

4. The method for predicting an amount of hydrogen generated in a rolling device according to claim 1 or 2, wherein the metal contact ratio is calculated by an impedance method.

5. The method for predicting an amount of hydrogen generated in a rolling device according to claim 1 or 2, wherein the exposed area of the newly-generated surface is calculated based on the following Formula (X), and a width of the actual contact surface is represented by the following Formula (Y),

$$\text{exposed area of newly-generated surface} = D \times V \times t... \quad (X)$$

wherein D represents a width (m) of the actual contact surface at an apparent contact surface between the sliding members, V represents a sliding speed (m/s), and t represents a contact time (s), and

$$\text{width of actual contact surface} = 2 \times (\alpha \times Aa/\pi)^{1/2}... \quad (Y)$$

wherein Aa represents an area ($m^2$) of the apparent contact surface, and $\alpha$ represents the metal contact ratio.

6. The method for predicting an amount of hydrogen generated in a rolling device according to claim 1 or 2, wherein the metal contact ratio $\alpha$ is represented by the following Formula (3),

$$\alpha = Ar/Aa... \quad (3)$$

wherein Ar represents an area ($m^2$) of the actual contact surface.

7. A hydrogen generation test device capable of, while causing two sliding members to slide against each other in a sealed container, measuring an amount of hydrogen generated from a lubricant interposed between at least the two sliding members, the hydrogen generation test device comprising:

   an impedance measuring device configured to measure an impedance between the two sliding members, wherein
   the two sliding members are capable of changing a sliding speed between the two sliding members,
   the impedance is used to calculate a metal contact ratio between the two sliding members, and
   the metal contact ratio and the sliding speed are used to calculate an exposed area of a newly-generated surface due to direct contact between the two sliding members.

8. The hydrogen generation test device according to claim 7, further comprising:

   a heater disposed in the sealed container and configured to heat the lubricant; and
   a temperature detection device configured to detect a temperature of the lubricant.

9. The hydrogen generation test device according to claim 8, further comprising:

   a gas port provided in the sealed container and configured to suck a gas containing hydrogen stored in a head space of the sealed container; and
   a hydrogen generation amount measuring device connected to the gas port through a tube and configured to measure an amount of hydrogen generated.

10. A method for evaluating likelihood of white structure peeling occurring in a rolling device, the method comprising:

   a hydrogen generation amount measurement step in which, while two sliding members are caused to slide against each other over a contact surface with a lubricant interposed between the two sliding members, an amount of hydrogen generated from the lubricant is measured;
   a threshold determination step in which a threshold of an amount of hydrogen generated in a case where white structure peeling is formed on the sliding members is determined based on the amount of hydrogen generated;
   a hydrogen generation amount prediction step in which an amount of hydrogen generated in the rolling device using the lubricant is predicted; and
   an evaluation step in which likelihood of white structure peeling occurring in the rolling device is evaluated by comparing the threshold of the amount of hydrogen generated with the amount of hydrogen generated that has been predicted in the hydrogen generation amount prediction step, wherein
   the hydrogen generation amount prediction step is performed by the method for predicting an amount of hydrogen generated in a rolling device according to claim 1 or 2.

*Fig.1*

## Fig.2

Fig.3

**Fig.4**

Fig.5

## Fig.6

# *Fig.7*

# Fig.8

Fig.9

## INTERNATIONAL SEARCH REPORT

| International application No. |
| --- |
| **PCT/JP2023/031576** |

| A. | CLASSIFICATION OF SUBJECT MATTER |
| --- | --- |

*G01N 17/00*(2006.01)i; *G01N 3/56*(2006.01)i; *G01M 13/04*(2019.01)i; *G01N 33/30*(2006.01)i
FI:   G01N17/00; G01N3/56 M; G01M13/04; G01N33/30

According to International Patent Classification (IPC) or to both national classification and IPC

| B. | FIELDS SEARCHED |
| --- | --- |

Minimum documentation searched (classification system followed by classification symbols)

G01N17/00; G01N3/56; G01M13/04; G01N33/30

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | WO 2020/110593 A1 (NSK LTD.) 04 June 2020 (2020-06-04)<br>paragraphs [0067]-[0082], fig. 3 | 1-10 |
| A | WO 2022/054352 A1 (NSK LTD.) 17 March 2022 (2022-03-17)<br>paragraphs [0014]-[0017], fig. 1 | 4-9 |
| A | WO 2012/117970 A1 (NTN CORP.) 07 September 2012 (2012-09-07)<br>entire text, all drawings | 1-10 |
| A | WO 2017/188314 A1 (NSK LTD.) 02 November 2017 (2017-11-02)<br>entire text, all drawings | 9 |
| A | US 2014/0157874 A1 (STRANDELL, Ingemar) 12 June 2014 (2014-06-12)<br>entire text, all drawings | 1-10 |
| A | X.Z. Liang. Hydrogen-accelerated white etching area formation in bearings under rolling contact fatigue. International Journal of Fatigue. June 2022, 159; 106753, pp. 1-6, Internet: <URL: https://doi.org/10.1016/j.ijfatigue.2022.106753>, entire text, all drawings<br>entire text, all drawings | 10 |

☑ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **31 October 2023** | **14 November 2023** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT

International application No.

**PCT/JP2023/031576**

### C.    DOCUMENTS CONSIDERED TO BE RELEVANT

| Category* | Citation of document, with indication, where appropriate, of the relevant passages | Relevant to claim No. |
|---|---|---|
| A | A. D. Richardson. Thermal Desorption Analysis of Hydrogen in Non-hydrogen-Charged Rolling Contact Fatigue-Tested 100Cr6 Steel. Tribology Letters. 2018, 66:4, pp. 1-16, Internet: <URL: https://doi.org/10.1007/s11249-017-0947-0>, entire text, all drawings<br>entire text, all drawings | 10 |
| A | 東根泰葉. 自動車用エンジン電装品・補機軸受のはく離について. ENEOS Technical Review. May 2009, vol. 51, no. 2, pp. 53-57, pp. 55, 56, [3.3. Vacuum Cutting Test or Slide Test], non-official translation (HIGASHINE, Yasuha. Detachment of Automotive Engine Electrical Components/Auxiliary Bearings.)<br>pp. 55, 56, [3.3. Vacuum Cutting Test or Slide Test] | 1-10 |
| P, A | WO 2022/270248 A1 (NSK LTD.) 29 December 2022 (2022-12-29)<br>entire text, all drawings | 1-10 |

Form PCT/ISA/210 (second sheet) (January 2015)

**INTERNATIONAL SEARCH REPORT**
Information on patent family members

International application No.

**PCT/JP2023/031576**

| Patent document cited in search report | | | Publication date (day/month/year) | Patent family member(s) | | | Publication date (day/month/year) |
|---|---|---|---|---|---|---|---|
| WO | 2020/110593 | A1 | 04 June 2020 | US | 2021/0318227 | A1 | |
| | | | | paragraphs [0094]-[0112], fig. 3 | | | |
| | | | | EP | 3889569 | A1 | |
| | | | | CN | 112639430 | A | |
| WO | 2022/054352 | A1 | 17 March 2022 | CN | 116113772 | A | |
| | | | | paragraphs [0065]-[0068], fig. 1 | | | |
| | | | | KR | 10-2023-0048419 | A | |
| WO | 2012/117970 | A1 | 07 September 2012 | EP | 2682732 | A1 | |
| | | | | entire text, all drawings | | | |
| | | | | CN | 103460009 | A | |
| WO | 2017/188314 | A1 | 02 November 2017 | US | 2019/0086382 | A1 | |
| | | | | entire text, all drawings | | | |
| | | | | EP | 3450816 | A1 | |
| | | | | CN | 108884965 | A | |
| US | 2014/0157874 | A1 | 12 June 2014 | WO | 2013/012364 | A1 | |
| | | | | CN | 103748451 | A | |
| WO | 2022/270248 | A1 | 29 December 2022 | (Family: none) | | | |

Form PCT/ISA/210 (patent family annex) (January 2015)

**EP 4 585 904 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013234883 A